## Europäisches Patentamt
## European Patent Office
## Office européen des brevets

(19)

(11) Veröffentlichungsnummer: **0 180 920 B1**

(12) **EUROPÄISCHE PATENTSCHRIFT**

(45) Veröffentlichungstag der Patentschrift: **02.01.92**

(51) Int. Cl.⁵: **C07K 1/00**, C07K 3/00, C07K 15/00

(21) Anmeldenummer: **85113857.8**

(22) Anmeldetag: **31.10.85**

(54) Verfahren zur Spaltung von Peptiden und Proteinen an der Methionyl-Bindung.

(30) Priorität: **09.11.84 DE 3440988**

(43) Veröffentlichungstag der Anmeldung:
**14.05.86 Patentblatt 86/20**

(45) Bekanntmachung des Hinweises auf die
Patenterteilung:
**02.01.92 Patentblatt 92/01**

(84) Benannte Vertragsstaaten:
**AT BE CH DE FR GB IT LI LU NL SE**

(56) Entgegenhaltungen:

**ADV. PROT. CHEM., Band 16, 1961, Seiten
221-321; B. WITKOP: "Nonenzymatic methods
for the preferential and selective cleavage
and modification of proteins"**

(73) Patentinhaber: **HOECHST AKTIENGESELL-
SCHAFT
Postfach 80 03 20
W-6230 Frankfurt am Main 80(DE)**

(72) Erfinder: **Bicker, Richard, Dr.
Brunnenstrasse 40
W-6237 Liederbach(DE)**
Erfinder: **Seipke, Gerhard, Dr.
Brunnenweg 4
W-6200 Wiesbaden(DE)**

EP 0 180 920 B1

## Beschreibung

Im Rahmen gentechnologischer Gewinnung von Peptiden und Proteinen (z.B. Insulin, Proinsulin, Preproinsulin und dessen Analoga, Interferon, Wachstumshormon) hat es sich oftmals als vorteilhaft erwiesen, die Produkte zunächst in der Form höhermolekularer "Fusionsproteine" zu isolieren.

Die so anfallenden Fusionsproteine sind stabiler als die gewünschten Produkte und können unter Ausnutzung von Induktionssystemen in höheren Ausbeuten isoliert werden. Für derartige Fusionen kommen im Prinzip alle in größeren Mengen von den Wirtsorganismen synthetisierten Proteine in Frage, vor allem wenn deren Synthese gut induzierbar ist; häufiger beschrieben sind die β-Galactosidase, β-Lactamase, Teile des Cro-Proteins, des C$_{II}$-Proteins und der Enzyme des Tryptophan-Stoffwechsels ( vor allem Trp D, Trp E). Dabei erfreuen sich Fusionen des gewünschten Genprodukts mit β-Galaktosidase besonderer Beliebtheit, weil die dabei zunächst anfallenden Proteine häufig schwerlöslich und somit gut anzureichern sind (A.V. Fowler und I. Zabin, J. Biol. Chem. 258 (1983) 14354-58).

Aus den resultierenden Fusionsproteinen können die gewünschten Produkte mit chemischen und enzymatischen Methoden abgespalten werden, wobei diese Spaltreaktionen hoch spezifisch sein müssen, um Schädigungen der Produkte zu vermeiden.

Chemische Methoden zur Proteinspaltung sind meist in Zusammenhang mit der Peptid-Sequenzanalytik von Proteinen referiert (siehe z.B. Handbook of Protein Sequence Analyses, 2nd Edition, L.R. Croft, John Wiley a. Sons; B. Witkop, Advan. Prot. Chem. 16 (1961), 221 sowie E. Gross, Methods in Enzymology, Vol. XI, 238 (1967)).

Besonders häufig wird eine Spaltung an Methionyl-Bindungen mit Bromcyan referiert (B. Witkop, Adv. Prot. Chem. 16 (1961) 221), da hiermit im allgemeinen hohe Spaltausbeuten ohne Schädigung anderer Peptidbindungen oder Aminosäureseitenketten erzielt werden.

Um hohe Spaltausbeuten zu erzielen, werden üblicherweise hohe Bromcyanüberschüsse (bis 250fach; siehe B. Witkop-Artikel) bezogen auf die zu spaltenden Methionyl-Bindung eingesetzt, bei langen Reaktionszeiten bis 30 Stunden und Raumtemperatur. Die Reaktion wird in stark saurem Milieu durchgeführt, ein bevorzugtes Lösungsmittel ist Ameisensäure im Konzentrationsbereich von 70-88 Volumen-%.

Bromcyan (BrCN) ist ein Feststoff (Schmp. 52°C, Kp. 62°C), eine hochtoxische Verbindung. Die tödliche Dosis für einen normaler Menschen beträgt 92 ppm bei einer Einwirkzeit von 10 Minuten (Gmelin Syst. Nr. 14 C, Fl. D 3 1976, S. 217 - 241; Petty's Industrial Hygiene and Toxicologiy, 3. Auflage 1982, Bd. 2 c, S. 4861). Bei unsauberen Präparaten ist ein spontaner Zerknall von Packungen beschrieben (s. Broschüre Sicherheit mit Merck, E. Merck, Darmstadt 5/13090/60/1282 R).

Die Handhabung großer Mengen BrCN bei der großtechnischen Proteinspaltung ist daher sehr problematisch, neben den Sicherheitsaspekten spielen auch technische Fragestellungen (z.B. Förderung von Feststoffen) eine Rolle.

Es wurde nun überraschenderweise gefunden, daß sich die Methionyl-Bindung mit Chlorcyan (ClCN) spalten läßt.

Die Erfindung betrifft daher ein Verfahren zur Spaltung von Peptiden und Proteinen an der Methionyl-Bindung, das dadurch gekennzeichnet ist, daß man die Spaltung mit Chlorcyan durchführt.

Proteinspaltungen mit ClCN sind in der Literatur nicht beschrieben.

Für den Fachmann ist der Einsatz von ClCN zur nichtenzymatischen Proteinspaltung nicht unbedingt vorhersehbar, da ClCH eine von BrCN deutlich unterschiedliche chemische Reaktivität zeigt (vgl. hierzu Houben-Weyl, Bd. II, S. 545; Ullmann Bd. 9, S. 668, B.S. Thyagarajan, The Chemistry of Cyanogen Halides 2, (1968); R.T. Parfitt, J. Chem. Soc. (C), 140, (1967)). Neben der unterschiedlichen Reaktivität und Aktivität von BrCN und ClCN beobachtet man auch unterschiedliches Reaktionsverhalten gegenüber gleichen Reaktionspartnern.

ClCN ist bei Raumtemperatur ein Gas (Schmp. -6,9°C; Sdp. 13,0°C); es ist nicht so toxisch wie BrCN (vgl. Gmelin Syst. Nr. 14 C, F 1, D 3, 1976, S. 185; Petty's Industrial Hygiene and Toxicology, 3. Auflage 1982, Bd. 2 c, S. 4859). Die tödliche Dosis für einen Menschen beträgt 159 ppm bei einer Einwirkzeit von 10 Minuten (vgl. 92 ppm für BrCN). Da der Reiz-Schwellenwert von ClCN mit 2,5 mg/m³ deutlich niedriger liegt als der von BrCN (6,0 mg/m³), hat ClCN eine größere "Selbstwarnung". Es besitzt daher für einen technischen Einsatz erhebliche Sicherheitsvorteile.

Daneben bietet der Einsatz von ClCN auch erhebliche verfahrenstechnische Vorteile: ClCN ist als Gas einfacher und gefahrloser dosierbar und förderbar als der Feststoff BrCN.

Nach beendeter Reaktion kann der Chlorcyan-Überschuß durch Einblasen eines geeigneten Gases aus dem Reaktionsgemisch entfernt werden. Geeignet sind inerte Gase wie z.B. Stickstoff. Zur Unterdrückung der Schaumbildung können geeignete Entschäumer zugesetzt werden. Gut geeignet sind z.B. die Typen SE 3, 6 und 9 der Firma Wacker Chemie. Im Anschluß daran wird das Reaktionsgemisch in üblicher Weise aufgearbeitet.

Der mit ClCN beladene Gasstrom kann durch einen Wäscher geleitet werden, der mit wäßriger NaOH- und/oder NaOCl-Lösung gefüllt ist. Dabei wird das ClCN zu NaCl und NaOCN abgebaut (s. Gmelin Syst. Nr. 14 C, F 1, D 3, 1976, S. 184; Petty's Industrial Hygiene and Toxicology, 3. Auflage 1982, Bd. 2 c, S. 4859).

Durch diese Verfahrensweise kann das Spaltreagenz (ClCN) wesentlich sicherer entsorgt werden als das toxischere BrCN. Die Entsorgung ist damit zeitlich und räumlich getrennt von der Proteinisolierung, die nach bekannten Methoden (Eindampfen, Gefriertrocknung) durchgeführt werden kann.

Chlorcyan wird vorteilhaft in einem 2 bis 30-fachen molaren Überschuß pro zu spaltende Methionyl-Bindung eingesetzt. Bevorzugt ist ein 5 bis 8-facher molarer Überschuß. Die Reaktionsdauer beträgt gewöhnlich 1-10 Stunden, vorzugsweise 3-6 Stunden.

Als Reaktionsmedium eignet sich ein Gemisch aus Wasser und einer Mineralsäure oder einer wassermischbaren organischen Säure. Bevorzugt sind organische Säuren, wie z.B. Ameisensäure. Der Gehalt von Ameisensäure im Reaktionsmedium kann 50-95 Volumen-%, vorzugsweise 65-88 Volumen-% betragen.

Die nachstehenden Beispiele dienen zur Erläuterung der Erfindung, ohne daß diese darauf beschränkt wäre.

## Beispiel 1 (Vergleichsbeispiel)

1 g $\beta$-Galactosidase aus E.coli (Gehalt ca. 70 %, Methioningehalt 0,16 mMol/g bestimmt durch Aminosäureanalyse) werden in 7,5 ml 70 Vol.-% HCOOH eingerührt und mit 0,135 g Bromcyan (= 1,28 mMol) versetzt.

Das Reaktionsgemisch wird 6 Stunden bei Raumtemperatur gerührt, mit 100 ml $H_2O$ verdünnt und gefriergetrocknet.

Der Rückstand beträgt 0,97 g Spaltproteingemisch.

In der Gelelektrophorese (SDS-PAGE) ist keine $\beta$-Galactosidase mehr zu erkennen. Man findet vorzugsweise Proteinfragmente im Molekulargewichtsbereich von 5 000 - 20000 Dalton. Nach Aminosäureanalyse und gaschromatographischer Methylrhodanidbestimmung beträgt die Spaltungsrate 94 %.

## Beispiel 2

1 g $\beta$-Galactosidase aus E.coli (Gehalt ca. 70 %, Methioningehalt 0,16 mMol/g bestimmt durch Aminosäureanalyse) werden in 7,5 ml 70 Vol.-% HCOOH eingerührt und mit 0,079 g ClCN (= 1,28 mMol) versetzt.

Das Reaktionsgemisch wird 6 Stunden bei Raumtemperatur gerührt.

Nach beendeter Reaktion werden 1-5 $\mu$l Entschäumer (SE 9 der Firma Wacker Chemie, Burghausen) zugesetzt und das überschüssige Chlorcyan durch Einblasen von $N_2$ (15 l/h) in ca. 1,5 Stunden entfernt. Der mit Chlorcyan beladene $N_2$-Strom wird in eine mit Natronlauge gefüllte Waschflasche eingeleitet.

Nach der ClCN-Austreibung wird die Lösung mit 100 ml $H_2O$ verdünnt und gefriergetrocknet. Der Rückstand beträgt 1 g.

In der SDS-PAGE ist keine $\beta$-Galactosidase mehr zu erkennen. Man findet vorzugsweise Proteinfragmente im Molekulargewichtsbereich von 5 000 - 20 000 Dalton. Nach Aminosäureanalyse und gaschromatographischer Methylrhodanidbestimmung beträgt die Spaltungsrate 93 %.

## Beispiel 3

1 g $\beta$-Lactamase, isoliert aus E.coli-Zellen, die das Plasmid pBR 322 enthalten (Gehalt ca. 75 %, Methioningehalt 0,28 mMol/g) werden in 10 ml 70 Vol.-% HCOOH eingerührt und mit 0,236 g ClCN (= 2,28 mMol) versetzt.

Das Reaktionsgemisch wird 6 Stunden bei Raumtemperatur gerührt.

Nach beendeter Reaktion werden 1-5 $\mu$l Entschäumer (SE 9 der Firma Wacker Chemie, Burghausen) zugesetzt und das überschüssige Chlorcyan durch Einblasen von $N_2$ (15 l/h) in ca. 1,5 Stunden entfernt. Der mit Chlorcyan beladene $N_2$-Strom wird in eine mit Natronlauge gefüllte Waschflasche eingeleitet.

Nach der ClCN-Austreibung wird die Lösung mit 100 ml $H_2O$ verdünnt und gefriergetrocknet.

Der Rückstand beträgt 0,99 g.

In der SDS-PAGE ist keine $\beta$-Lactamase mehr zu erkennen. Man findet vor allem Proteinfragmente im Molekulargewichtsbereich 3 000 - 10 000 Dalton. Nach Aminosäureanalyse und gaschromatographischer Methylrhodanidbestimmung beträgt die Spaltungsrate 92 %.

## Patentansprüche

1. Verfahren zur Spaltung von Peptiden und Proteinen an der Methionyl-Bindung, dadurch gekennzeichnet, daß man die Spaltung mit Chlorcyan durchführt.

2. Verfahren gemäß Anspruch 1, dadurch gekennzeichnet, daß man nach beendeter Reaktion den Chlorcyan-Überschuß mit einem geeigneten Gas aus dem Reaktionsgemisch entfernt und das Reaktionsgemisch anschließend in üblicher Weise aufarbeitet.

3. Verfahren gemäß Anspruch 2, dadurch gekennzeichnet, daß man den Chlorcyan-Überschuß mit Stickstoff aus dem Reaktionsgemisch entfernt.

4. Verfahren gemäß einem der Ansprüche 1-3, dadurch gekennzeichnet, daß man Chlorcyan in einem 2 bis 30-fachen molaren Überschuß pro zu spaltende Methionyl-Bindung einsetzt.

5. Verfahren gemäß einem der Ansprüche 1-4, dadurch gekennzeichnet, daß man Chlorcyan in einem 5 bis 8-fachen molaren Überschuß pro zu spaltende Methionyl-Bindung einsetzt.

6. Verfahren gemäß einem der Ansprüche 1-5, dadurch gekennzeichnet, daß man die Reaktion in 1-10 Stunden durchführt.

7. Verfahren gemäß einem der Ansprüche 3-6, dadurch gekennzeichnet, daß man die Reaktion in 3-6 Stunden durchführt.

8. Verfahren gemäß einem der Ansprüche 3-7, dadurch gekennzeichnet, daß man als Reaktionsmedium ein Gemisch aus Wasser und einer wassermischbaren Säure verwendet.

9. Verfahren gemäß Anspruch 8, dadurch gekennzeichnet, daß man als Reaktionsmedium ein Gemisch aus Wasser und Ameisensäure verwendet.

10. Verfahren gemäß Anspruch 9, dadurch gekennzeichnet, daß der Ameisensäure-Gehalt im Reaktionsmedium 50-95 Volumen-% beträgt.

## Claims

1. A process for the cleavage of peptides and proteins at the methionyl bond, which comprises carrying out the cleavage with cyanogen chloride.

2. The process as claimed in claim 1, wherein the excess cyanogen chloride is removed, after the reaction is complete, from the reaction mixture using a suitable gas, and the reaction mixture is then worked up in a customary manner.

3. The process as claimed in claim 2, wherein the excess cyanogen chloride is removed from the reaction mixture using nitrogen.

4. The process as claimed in one of claims 1-3, wherein cyanogen chloride is used in a 2 to 30-fold molar excess per methionyl bond which is to be cleaved.

5. The process as claimed in one of claims 1-4, wherein cyanogen chloride is used in a 5 to 8-fold molar excess per methionyl bond which is to be cleaved.

6. The process as claimed in one of claims 1-5, wherein the reaction is carried out in 1-10 hours.

7. The process as claimed in one of claims 3-6, wherein the reaction is carried out in 3-6 hours.

8. The process as claimed in one of claims 3-7, wherein the reaction medium used is a mixture of water and an acid which is miscible with water.

9. The process as claimed in claim 8, wherein the reaction medium used is a mixture of water and formic acid.

10. The process as claimed in claim 9, wherein the formic acid content in the reaction medium is 50-95% by volume.

## Revendications

1. Procédé pour la coupure de peptides et de protéines au niveau de la liaison méthionyle, caractérisé en ce que l'on effectue la coupure à l'aide de chlorure de cyanogène.

2. Procédé selon la revendication 1, caractérisé en ce que, une fois la réaction terminée, on élimine hors du mélange réactionnel l'excès de chlorure de cyanogène à l'aide d'un gaz approprié, et on achève ensuite le traitement du mélange réactionnel de la façon usuelle.

3. Procédé selon la revendication 2, caractérisé en ce que l'on élimine hors du mélange réactionnel l'excès de chlorure de cyanogène à l'aide d'azote.

4. Procédé selon l'une des revendications 1 à 3, caractérisé en ce que l'on utilise du chlorure de cyanogène en un excès de 2 à 30 fois molaire, par liaison méthionyle à couper.

5. Procédé selon l'une des revendications 1 à 4, caractérisé en ce que l'on utilise du chlorure de cyanogène en un excès de 5 à 8 fois molaire par liaison méthionyle à couper.

6. Procédé selon l'une des revendications 1 à 5, caractérisé en ce que l'on effectue la réaction en 1-10 heures.

7. Procédé selon l'une des revendications 3 à 6, caractérisé en ce que l'on effectue la réaction en 3-6 heures.

8. Procédé selon l'une des revendications 3 à 7, caractérisé en ce que l'on utilise en tant que milieu réactionnel un mélange d'eau et d'un acide miscible à l'eau.

9. Procédé selon la revendication 8, caractérisé en ce que l'on utilise en tant que milieu réactionnel un mélange d'eau et d'acide formique.

10. Procédé selon la revendication 9, caractérisé en ce que la teneur du milieu réactionnel en acide formique va de 50 à 95 % en volume.